# EUROPEAN PATENT SPECIFICATION

(11) **EP 4 115 880 B1**
(45) Date of publication and mention of the grant of the patent: **22.07.2026**
(21) Application number: 21760398.4
(22) Date of filing: 26.02.2021
(51) Int. Cl.: A61K 31/145, A61P 31/14, A61P 11/00

(54) **DISULFIRAM FOR USE IN THE TREATMENT AND/OR PROPHYLAXIS OF A DISEASE CAUSED BY SARS-COV-2**
DISULFIRAM ZUR VERWENDUNG BEI DER BEHANDLUNG UND/ODER PROPHYLAXE VON DURCH SARS-COV-2 VERURSACHTEN KRANKHEITEN
DISULFIRAM POUR UTILISATION DANS LE TRAITEMENT ET/OU LA PROPHYLAXIE D'UNE MALADIE CAUSÉE PAR LE SARS-COV-2

(30) Priority: 26.02.2020 CN 202010121688
(43) Date of publication of application: 11.01.2023
(73) Proprietor: ShanghaiTech University, Shanghai 201210 (CN)
(72) Inventor: YANG, Haitao, Shanghai 201210 (CN); JIN, Zhenming, Shanghai 201210 (CN); YANG, Xiuna, Shanghai 201210 (CN); ZHAO, Yao, Shanghai 201210 (CN); RAO, Zihe, Shanghai 201210 (CN)
(74) Representative: Greaves Brewster LLP
(86) International application number: PCT/CN2021/078185
(87) International publication number: WO 2021/170093

(56) References cited:
- WO-A1-2021/234362
- CN-A- 1 763 002
- CN-A- 103 301 097
- US-A1- 2013 203 715
- US-A1- 2017 364 630
- JIN ZHENMING ET AL: "Structure of Mfrom SARS-CoV-2 and discovery of its inhibitors", NATURE,, vol. 582, no. 7811, 9 April 2020 (2020-04-09), pages 289 - 293, XP037163556, DOI: 10.1038/S41586-020-2223-Y
- TAMBURIN STEFANO ET AL: "COVID-19 and related symptoms in patients under disulfiram for alcohol use disorder", INTERNAL AND EMERGENCY MEDICINE, SPRINGER MILAN, MILAN, vol. 16, no. 6, 19 January 2021 (2021-01-19), pages 1729 - 1731, XP037534924, ISSN: 1828-0447, [retrieved on 20210119], DOI: 10.1007/S11739-021-02633-Y
- LIANG RUIYING, WANG LILI, ZHANG NARU, DENG XIAOQIAN, SU MENG, SU YUDAN, HU LANFANG, HE CHEN, YING TIANLEI, JIANG SHIBO, YU FEI: "Development of Small-Molecule MERS-CoV Inhibitors", VIRUSES, vol. 10, no. 12, 17 December 2018 (2018-12-17), XP055841380, DOI: 10.3390/v10120721
- LIN MIN-HAN, MOSES DAVID C., HSIEH CHIH-HUA, CHENG SHU-CHUN, CHEN YAU-HUNG, SUN CHIAO-YIN, CHOU CHI-YUAN: "Disulfiram can inhibit MERS and SARS coronavirus papain-like proteases via different modes", ANTIVIRAL RESEARCH, ELSEVIER BV, NL, vol. 150, 1 February 2018 (2018-02-01), NL, pages 155 - 163, XP055823265, ISSN: 0166-3542, DOI: 10.1016/j.antiviral.2017.12.015

## Description

The present application claims priority of Chinese Patent Application No. 2020101216884 filed on Feb. 26, 2020.

### Technical Field

The present invention belongs to the technical field of biomedicines, relates to use of disulfiram in resisting coronavirus, and particularly relates to use of disulfiram in the preparation of a medicament for the treatment and/or prophylaxis of a disease caused by coronavirus.

### Background

Coronaviruses are a group of viruses closely related to humans and livestock. The coronaviruses HCoV-229E and HCoV-OC43 will cause the common cold (van der Hoek, L., Pyrc, K., Jebbink, M. et al., Identification of a new human coronavirus., Nat Med, 2004,10, 368-373*).* Severe acute respiratory syndrome (SARS) caused by SARS coronavirus from 2002 to 2003 has infected 8098 people worldwide (Stadler, K., Masignani, V, Eickmann, M. et al. SARS - beginning to understand a new virus., Nat Rev Microbiol, 2003, 1, 209 218*).* It was identified in 2004 that HCoV-NL63 may also cause cold-like respiratory diseases (van der Hoek, L., Pyrc, K., Jebbink, M. et al., Identification of a new human coronavirus., NatMed, 2004,10, 368-373). The middle east respiratory syndrome coronavirus (MERS-CoV) appeared in 2012 had infected 1,728 people in 27 countries by Apr. 26, 2016 (*de* Wit, E., van Doremalen, N., Falzarano, D. et al., SARS and MERS: recent insights into emerging coronaviruses. Nat Rev Microbiol, 2016,14, 523-534*.*). The newly popular SARS-CoV-2 virus will cause COVID-19 with clinical manifestations of fever, dry cough and dyspnea *(*Jeannette Guarner, MD, Three Emerging Coronaviruses in Two Decades: The Story of SARS, MERS, and Now COVID-19, American Journal of Clinical Pathology, aqaa029)*.* Coronaviruses have a great impact on animal husbandry: porcine epidemic diarrhea virus (PEDV),transmissible gastroenteritis virus (TGEV) and porcine delta coronavirus (PDCoV, also called Deltavirus) may cause severe enteritis, diarrhea, vomiting and dehydration in pigs, bringing huge losses to the pig farming industry (Akimkin V, Beer M, Blome S, et al. New Chimeric Porcine Coronavirus in Swine Feces, Germany, 2012., Emerg Infect Dis., 2016, 22(7):1314-1315). Feline infectious peritonitis virus (FIPV) may cause fatal diseases in felines. Avian infectious bronchitis virus (IBV), a widely distributed disease in poultry, infects poultry and has a tremendous negative impact on the poultry industry.

Coronaviruses belong to the *Orthocoronavirinae* subfamily of the *Coronaviridae* family in the *Nidovirales* order. The genome sequence of the SARS-CoV-2 coronavirus has been published, and the nucleotide similarity of the sequence to SARS-CoV is about 90%, and the sequence similarity of the protein sequence to SARS-CoV is about 80%. The genome of coronaviruses is a single-stranded positive-stranded RNA with a length of approximately 28 kb, and encodes primarily structural proteins required for viral packaging and non-structural proteins associated with replication and transcription. The development of medicaments and vaccines for the treatment of coronaviruses-related diseases is mainly directed to the above two types of proteins. Two thirds of the genes of the viral genome encode primarily non-structural proteins. The virus encodes two polymerase proteins, that is pp1a and pp1ab, which are involved in the viral replication process. Pp1a and pp1ab can be cut into 16 non-structural proteins (nsp1-16) by two kinds of virus-encoded papain-like protease and main protease. Only when these functional subunits are cut into separate protein units by virus-encoded protease, can the virus complete normal transcription and replication functions, and further assemble into replication-transcription complexes to complete the replication and transcription of viruses. Wherein, the papain-like protease has 3 restriction sites; the main protease has 11 restriction sites at pp1a and pp1ab. It can be seen that the main protease plays a key regulatory role in virus transcription and replication, and thus has been the focus of research (Ziebuhr, J.; Snijder, E. J.; Gorbalenya, A. E. Virus-encoded proteinases and proteolytic processing in the Nidovirales. J Gen Virol, 2000, 81, 853-79*.;* Ziebuhr, J. Molecular biology of severe acute respiratory syndrome coronavirus. Curr Opin Microbiol, 2004, 7, 412-9*.*). Because of the importance of the main protease to the amplification and replication of coronaviruses, it is particularly important to find an inhibitor with strong specificity and high safety for the catalytic site of the main protease for medicament development.

Disulfiram (CAS No.: 97-77-8) is a specific inhibitor of acetaldehyde dehydrogenase (ALDH 1) (Liu, Xinwei, et al., "Targeting ALDH1A1 by disulfiram/copper complex inhibits non-small cell lung cancer recurrence driven by ALDH-positive cancer stem cells. " Oncotarget 7.36 (2016): 58516.) and is used to treat chronic alcoholism and acute sensitivity to alcohol. Disulfiram also acts on the dopaminergic system, and both disulfiram and a metabolite thereof, carbon disulfide, can inhibit dopamine-hydroxylase (DBH) and increase dopamine levels. This may lead to neuropsychiatric symptoms such as delirium, paranoid, memory disorders, ataxia, dysarthria and frontal lobe release signs. In addition to this effect, disulfiram can also inhibit dopamine-hydroxylase, leading to the increased dopamine concentration and the decreased norepinephrine concentration in the brain, which may suggest an anti-addictive effect of disulfiram in alcohol dependence.

Nature 2017 (Skrott, Zdenek, et al. "Alcohol-abuse drug disulfiram targets cancer via p97 segregase adaptor NPL4. " Nature 552.7684 (2017): 194-199*.]*) published the anti-tumor activity of disulfiram, and it had been found that disulfiram can be metabolized into a small molecule in vivo, thereby promoting the coagulation of the native protein NPL4 and binding to its partner p97 enzyme, which inhibits the function of NPL4-p97 complex promoting the tumor growth, and further induces cancer cell death. In addition, disulfiram has also been found to have specific activity on zinc finger and ring finger ubiquitin E3 ligases which play an important role in the development of cancer (R Kona, F., D. A. M. B. Buac, and A. M Burger. "Disulfiram, and disulfiram derivatives as novel potential anticancer drugs targeting the ubiquitin proteasome system in both preclinical and clinical studies." Current cancer drug targets 11.3 (2011): 338-346.). A related patent application is WO2017077336A1.

*LIANG, R.Y. et al.* discloses disulfiram can be used as an allosteric inhibitor of MERS-CoV papain (LIANG, R.Y. et al. Development of Small-Molecule MERS-CoV Inhibitors. Virus. 17.12.2018). *LIN, M.H. et al.* discloses that disulfiram inhibits MERS-CoV PL^{pro}, SARS-CoV PL^{pro}s and SARS-CoV PL^{pro} *in vitro* (LIN, MH. et al. Disulfiram can inhibit MERS and SARS coronavirus papain-like protease via different modes. Antiviral Research. 28.12.2017).

### Content of the present invention

The invention is set out in the appended set of claims.

To solve the technical problem that there is no medicament for the effective treatment and/or prophylaxis of a disease caused by coronavirus in the prior art, the present invention provides disulfiram for use in the treatment and/or prophylaxis of a disease caused by SARS-CoV-2.

The present invention mainly solves the technical problem through the following technical solutions.

The present invention provides use of disulfiram (CAS No. 97-77-8) for use in the treatment and/or prophylaxis of a disease caused by SARS-CoV-2.

Preferably, the disease is a mammalian or avian disease, wherein the mammal includes humans and cats.

The coronaviruses described herein are defined as well known in the art and belong to the *Orthocoronavirinae* subfamily of the *Coronaviridae* family in the *Nidovirales* order. Coronaviruses are a wide class of viruses widely existing in nature with envelope and genome of a single-stranded positive-stranded RNA.

The coronaviruses described herein preferably belong to the *Orthocoronavirinae* subfamily, and more preferably belongs to genera of Alpha coronavirus, Beta coronavirus, Gamma coronavirus or Delta coronavirus.

In a preferred embodiment of the present invention, disulfiram can be used for the treatment of severe infectious diseases caused by other coronaviruses such as SARS-CoV (Betacoronavirus genus) and MERS-CoV in addition to the treatment of diseases caused by SARS-CoV-2 (Betacoronavirus genus), and it can also be used as a common cold medicament for the treatment of diseases caused by coronaviruses such as HCoV-HKU1 (*Human coronavirus,* HKU1; Betacoronavirus genus), HCoV-NL63 (*Human coronavirus,* NL63; Alphacoronavirus genus), HCoV-OC43 (*Human coronavirus*, OC43) and HCoV-229E (*Human coronavirus 22E*; Alphacoronavirus genus); it can also be used as a veterinary medicament for the treatment of animal diseases such as transmissible gastroenteritis virus (TGEV; Alphacoronavirus genus), porcine epidemic diarrhea virus (PEDV; Alphacoronavirus genus), porcine delta coronavirus (PDCoV; Deltacoronavirus genus), feline infectious peritonitis virus (FIPV; Alphacoronavirus genus), and infectious bronchitis virus (IBV; Gammacoronavirus genus).

Thus, the coronaviruses disclosed herein are preferably selected from SARS-CoV-2, SARS-CoV, MERS-CoV, HCoV-HKU1, HCoV-NL63, HCoV-OC43, HCoV-229E, TGEV, PEDV, PDCoV, FIPV and IBV.

In a certain embodiment, disulfiram or a pharmaceutically acceptable salt thereof disclosed herein is present in the form of a pharmaceutical composition comprising the same; preferably, carmofur and/or pharmaceutically acceptable salt thereof is a sole active ingredient of the pharmaceutical composition; and/or, the pharmaceutical composition further comprises a pharmaceutically acceptable carrier, such as a pharmaceutically acceptable auxiliary material.

In a certain embodiment, disulfiram or a pharmaceutically acceptable salt thereof disclosed herein is present in the form of a kit comprising the same, the kit further comprises a medicament for the treatment of a coronavirus-related disease and/or a medicament for the treatment of another disease caused by virus.

The pharmaceutical composition comprising disulfiram as an active ingredient in the present invention can be prepared according to methods known in the art. The compounds disclosed herein may be prepared into any dosage form suitable for human or animal use. The compounds disclosed herein are generally present in the pharmaceutical composition thereof in an amount of 0.1% to 99.0% by weight.

The pharmaceutically acceptable carrier may be a carrier conventional in the art, and the carrier may be any suitable physiologically or pharmaceutically acceptable auxiliary material. The pharmaceutically acceptable auxiliary material is conventional in the art, and preferably comprises a pharmaceutically acceptable excipient, a filler, a diluent, or the like. More preferably, the pharmaceutical composition comprises 0.01%-99.99% of the protein and/or the antibody-drug conjugate and 0.01%-99.99% of a pharmaceutically acceptable carrier, the percentage being the mass percentage of the pharmaceutical composition.

Disulfiram or the pharmaceutical composition comprising the same disclosed herein can be administered in a unit dosage form, and the administration route can be intestinal or parenteral administration, such as oral, intravenous, intramuscular, subcutaneous, nasal, buccal, ophthalmic, intrapulmonary and respiratory, cutaneous, vaginal, rectal.

The dosage form for administration may be a liquid, solid or semi-solid dosage form. The liquid dosage form may be solutions (including true solutions and colloidal solutions), emulsions (including o/w type, w/o type and multiple emulsions), suspensions, injections (including water injections, powder for injections and infusions), eye drops, nasal drops, lotions, liniments, and the like; the solid dosage form may be tablets (including common tablets, enteric coated tablets, buccal tablets, dispersible tablets, chewable tablets, effervescent tablets, and orally disintegrating tablets), capsules (including hard capsules, soft capsules, and enteric coated capsules), granules, powders, pellets, dripping pills, suppositories, films, patches, aerosols (powder sprays), sprays, and the like; the semi-solid dosage form may be ointments, gels, pastes, and the like.

Disulfiram disclosed herein can be prepared into common preparations, and can also be prepared into sustained release preparations, controlled release preparations, targeting preparations and various particle drug delivery systems.

The term "pharmaceutically acceptable" means that the salts, solvents, auxiliary materials, etc., are generally non-toxic, safe, and suitable for use in a patient. The "patient" is preferably a mammal, and more preferably a human.

The term "pharmaceutically acceptable salt" refers to a salt of disulfiram disclosed herein prepared with a relatively non-toxic, pharmaceutically acceptable acid or base.

On the basis of the general knowledge in the art, the above preferred conditions can be combined arbitrarily to obtain preferred embodiments of the present invention.

The reagents and starting materials used in the present invention are commercially available.

The positive progressive effects of the present invention are as follows:

It is found in the present invention that disulfiram can well inhibit the activity main proteases of coronavirus in an *in-vitro* enzyme activity experiment, and thus the defect that the prior art is lack of treatment of diseases caused by coronaviruses is overcome. Disulfiram can be further researched and modified for application.

### Brief description of the drawings

FIG. 1: the inhibitory activity of disulfiram against the main protease of the novel coronavirus; in the figure, 1: DMSO (negative control), 2: compound molecule, and 3: positive control (N3 inhibitor).
FIG. 2: an inhibition curve of disulfiram against the main protease of the novel coronavirus.
FIG. 3:the inhibitory activity of disulfiram against the novel coronavirus at the cellular level.

### Detailed description of the preferred embodiment

The present invention is further illustrated by the following examples, which are not intended to limit the present invention. Experimental procedures without specified conditions in the following examples were performed in accordance with conventional procedures and conditions, or in accordance with instructions.

### Example 1

High-throughput screening was carried out in different compound libraries, and the successfully screened disulfiram showed a relatively good inhibition effect on the main protease of the novel coronavirus. The conditions of an enzyme activity experiment are as follows:

Fluorogenic substrate for enzyme activity assay: MCA-AVLQSGFR-Lys(Dnp)-Lys-NH₂, with an excitation wavelength and an emission wavelength of 320 nm and 405 nm, respectively.

The enzyme activity reaction buffer was 50 mM Tris-HCl (pH 7.3) and 1 mM EDTA; the enzyme activity reaction temperature was 30 °C. The reaction was initiated by adding protease at a final concentration of 0.2 µM, wherein the substrate concentration was 20 µM, and the concentration of disulfiram was 1.5 µM (Xue X, Yang H, Shen W, et al. Production of authentic SARS-CoV Mpro with enhanced activity: application as a novel tag-cleavage endopeptidase for protein overproduction[J]. Journal of molecular biology, 2007, 366(3): 965-975*.*).

The positive control used was N3 inhibitor, which is known to effectively inactivate the coronavirus main protease (Yang Haitao, Xie Weiqing, Xiaooyu X, et al., Design of wide-spectrum inhibitors targeting coronavirus main proteases [J]. Living organic chemistry, 2005, 3(10): 1742-1752), and the structural formula is shown below:

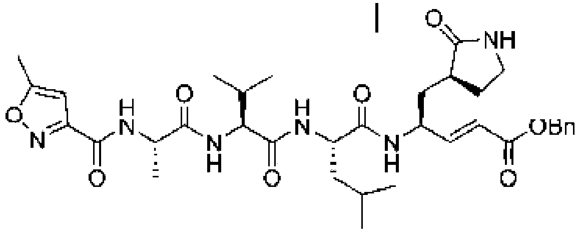

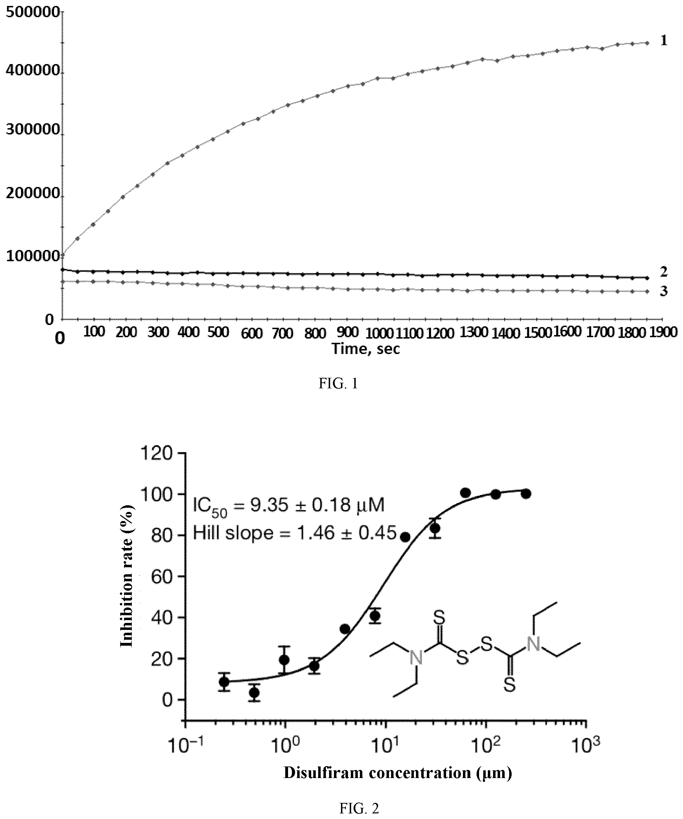

According to FIG. 1, it can be seen that the enzyme activity experimental curve of disulfiram is greatly different from that of negative control, and which means disulfiram has the significant inhibitory activity against the coronavirus main protease.

### Example 2

Fluorogenic substrate for determination of enzyme activity inhibition curve: MCA-AVLQSGFR-Lys(Dnp)-Lys-NH₂, with an excitation wavelength and an emission wavelength of 320 nm and 405 nm, respectively.

The buffer for enzyme activity inhibition curve determination was 50 mM Tris-HCl (pH 7.3) and 1 mMEDTA; the reaction temperature was 30 °C. The reaction was initiated by adding protease at a final concentration of 0.2 µM, wherein the substrate concentration was 20 µM; the determination was performed using 11 different concentrations of disulfiram, and the test results showed an IC₅₀ value of 9.35 µM (FIG. 2) *(*Jin Z, Du X, Xu Y, et al. Structure of Mpro from COVID-19 virus and discovery of its inhibitors[J]. Nature, 2020, 582(7811):1-9).

### Example 3

The *in-vitro* cell antiviral experiment adopted qRT-PCR method for detection. Approximately Vero cells were seeded into a 96-well plate and incubated at 37 °C for 20 h to 24 h before treating the cells with 10 µM disulfiram for 1 h. SARS-CoV-2 was then added to treat the cells for 2 h for infection, an MOI (multiplicity of infection) of 0.01 was observed. The virus-drug mixture was then removed, and after washing, the cells were further cultured in fresh media containing drugs. After 72-hour cultivation, viral RNA was extracted from the culture supernatant using an QIAamp viral RNA mini kit (Qiagen) and finally detected by qRT-PCR (Jin Z, Du X, Xu Y, et al. Structure of Mpro from COVID-19 virus and discovery of its inhibitors[J]. Nature, 2020, 582(7811):1-9).

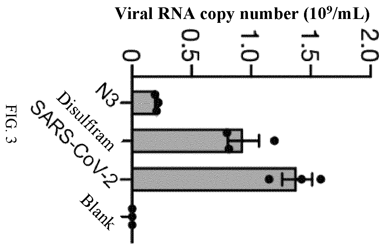

Disulfiram has been found to inhibit the replication of SARS-CoV-2 through an *in-vitro* cellular antiviral experiment, and thus disulfiram can be used for the treatment of diseases caused by coronavirus(FIG. 3).

Although specific embodiments of the present invention have been described above, it will be understood by those skilled in the art that these embodiments are merely illustrative and that many variations or modifications can be made to these embodiments without departing from the present invention. The scope of protection of the present invention is therefore defined by the appended claims.

## Claims

1. Disulfiram for use in the treatment and/or prophylaxis of a disease caused by SARS-CoV-2.

2. The disulfiram for use according to claim 1, wherein the disease is a mammalian or avian disease; wherein the mammal comprises human and cats.

3. The disulfiram for use according to claim 1 wherein disulfiram is a sole active ingredient of a pharmaceutical composition.

4. The disulfiram for use according to claim 3, wherein the pharmaceutical composition further comprises a pharmaceutically acceptable carrier.

5. The disulfiram for use according to claim 4, wherein the pharmaceutically acceptable carrier is a pharmaceutically acceptable auxiliary material.

6. The disulfiram for use according to any one of claims 1 to 5, wherein disulfiram is present in the form of a kit comprising the same, and the kit further comprises a medicament for the treatment of a coronavirus-related disease.

## Patentansprüche

1. Disulfiram zur Verwendung bei der Behandlung und/oder Prophylaxe einer durch SARS-CoV-2 verursachten Krankheit.

2. Disulfiram zur Verwendung nach Anspruch 1, wobei die Krankheit eine Säugetier- oder Vogelkrankheit ist; wobei das Säugetier Menschen und Katzen umfasst.

3. Disulfiram zur Verwendung nach Anspruch 1, wobei Disulfiram ein einzelner aktiver Inhaltsstoff einer pharmazeutischen Zusammensetzung ist.

4. Disulfiram zur Verwendung nach Anspruch 3, wobei die pharmazeutische Zusammensetzung ferner einen pharmazeutisch unbedenklichen Träger umfasst.

5. Disulfiram zur Verwendung nach Anspruch 4, wobei der pharmazeutisch unbedenkliche Träger ein pharmazeutisch unbedenkliches Hilfsmaterial ist.

6. Disulfiram zur Verwendung nach einem der Ansprüche 1 bis 5, wobei Disulfiram in der Form eines Kits vorliegt, das dasselbe umfasst, und das Kit ferner ein Medikament für die Behandlung einer Coronavirus-bedingten Krankheit umfasst.

## Revendications

1. Disulfirame destiné à être utilisé dans le traitement et/ou la prophylaxie d'une maladie causée par le SRAS-CoV-2.

2. Disulfirame destiné à être utilisé selon la revendication 1, ladite maladie étant une maladie de mammifère ou aviaire ; ledit mammifère comprenant l'être humain et les chats.

3. Disulfirame destiné à être utilisé selon la revendication 1, ledit disulfirame étant un principe actif unique d'une composition pharmaceutique.

4. Disulfirame destiné à être utilisé selon la revendication 3, dans lequel la composition pharmaceutique comprend en outre un support pharmaceutiquement acceptable.

5. Disulfirame destiné à être utilisé selon la revendication 4, dans lequel le support pharmaceutiquement acceptable est une matière auxiliaire pharmaceutiquement acceptable.

6. Disulfirame destiné à être utilisé selon l'une quelconque des revendications 1 à 5, ledit disulfirame étant présent sous la forme d'un kit le comprenant, et ledit kit comprenant en outre un médicament destiné au traitement d'une maladie liée à un coronavirus.
